# EUROPEAN PATENT APPLICATION

(11) **EP 2 374 526 A1**
(43) Date of publication of application: **12.10.2011**
(21) Application number: 10305320.3
(22) Date of filing: 29.03.2010
(51) Int. Cl.: B01D 71/02, B01D 67/00, B01D 53/22, B01J 23/00, B01J 23/10, B01J 23/22, B01J 23/58, B01J 35/06, B01J 37/02, B01D 69/12, C07C 2/84

(54) **Solid composite membrane exhibiting both oxygen conductivity and a substrate catalyst interface**

(71) Applicant: Centre National de la Recherche Scientifique (C.N.R.S), 75016 Paris (FR); UCBL Universite Claude Bernard De Lyon 1, 69622 Villeurbanne Cedex (FR)
(72) Inventor: Mirodatos, Claude, 69003 Lyon (FR); Olivier, Louis, 38200 Chuzelles-Les-Pins (FR); Van Veen, André Cornelis, 47661 Issum (DE); Haag, Stéphane, 68600 Volgelsheim (FR)
(74) Representative: Colombet, Alain André

(57) **Abstract**

The present invention relates to an oxygen permeable composite membrane comprising :
- a first dense perovskite membrane, and
- a porous perovskite membrane on one side of the dense perovskite membrane.

The present invention also relates to such membrane further comprising a catalyst layer on the porous membrane.
The invention also relates to production and use of such membrane, especially in OCM reaction.

## Description

The present invention relates to a new composite membrane for high temperature oxygen permeation, its method of preparation and use thereof.

The synthesis of C2 compounds (ethylene and ethane) is of great interest since such compounds, essentially ethylene, are important raw materials in chemistry, leading to polymers, plastics and synthetic fuel (diesel fuel, gasoline...).

Production of value-added chemicals, such as C2 hydrocarbons (ethane and ethylene), from natural resources is performed in industry via different processes. Ethane is often present in natural gas between 1 and 10%; it therefore can be extracted from gas by cryodistillation and thermal cracking. Ethylene is mainly produced by steam cracking in oil industry. All these processes are easy to implement, but are very energy-consuming. Moreover, taking into account the important reduction of petroleum resources, other methods have to be provided to furnish C2 hydrocarbons which would be less energy-consuming.

The valorisation of natural gases is a domain of permanent research, since resources in natural gases are much more important than that of petroleum. The abundant resources of natural gases, available at lower prices, have reactivated research on the oxidative coupling of methane (OCM) yielding ethane and ethylene, as value-added and easy-to-liquefy products.

In previous literature studies, assuming lower oil prices, it has been estimated that a single-pass conversion of 35-37% and selectivity of 88-85%, equivalent to a C2 yield greater than 30%, is required to attain commercial competitiveness for OCM.

The OCM reaction can be realized in a classical fixed-bed reactor. The main drawback is that the oxidation reaction may occur in gas phase instead of the catalyst surface and thus may lead to complete oxidation of methane to CO or CO₂ instead of C2 products. The limit of the C2 yields in fixed-bed reactors is around 25% which is far away from the required yield to attain commercial competitiveness.

Catalytic membrane reactors using solid-state membranes for the oxidation or decomposition of various chemical species have been studied and used previously to solve this problem. In catalytic membrane reactor, that facilitates partial oxidation reactions, a catalytic membrane separates an oxygen-containing gas, such as air, from a reactant gas to be oxidized. Oxygen or other oxygen-containing species, such as NO₂, are reduced at one face of the membrane to oxygen anions that are then transported across the membrane to its other face in contact with the reactant gas. The membrane is a gas-impermeable dense membrane that exhibits ionic conductivity. The reactor has an oxidation zone and a reduction zone separated by the membrane which itself has an oxidation surface exposed to the oxidation zone and a reduction surface exposed to the reduction zone. For OCM the reactant gas is a mixture of methane and an inert diluent, such as helium.

Materials for membranes in catalytic membrane reactors must have oxygen anion conductivity properties, and the materials must be chemically and mechanically stable at the high operating temperature (800-1200°C) and under the harsh conditions required.

Different studies were carried out on membrane allowing separation of gases, especially EP1 057 798, which describes a process for making a substantially cubic perovskite crystalline structure of at least one strontium-doped lanthanum cobalt oxide that comprises incorporating in the oxide a stabilizing amount of at least one cerium gadolinium oxide (CGO). It is underlined that this membrane may be used in processes where oxygen is to be separated from oxygen-containing gas streams at a temperature from about 400 to about 800°C. Thus this membrane could not be used in OCM which is carried out at more elevated temperature, i.e. from 800 to 1200 °C

Patent US 5,198,269 describes a sol-gel method for producing crystalline thin films of perovskite compounds with better crystallinity and on a wider variety of substrates by deposition of an intermediate perovskite film (interlayer) by dip-coating or spin-coating.

Patent US 4,828,585 describes a method of using an improved membrane to separate gas mixtures. The method consists of modifying the surface of a gas separating membrane by using gaseous fluorine or a combination of fluorine and sulphur dioxide. However, such a method, for improving the properties of a membrane which serves to separate gas mixture, is not really environmental-friendly and dangerous since it uses fluorine compounds, which especially could require specific equipment. Moreover, those compounds are not stable at high temperature for the coupling of methane. Finally, the acid functions introduced by those treatments would lead to lower properties.

US 5,648,304 describes novel mixed conductor membranes which are capable of separating oxygen from oxygen-containing feeds at elevated temperatures. The membranes have a substantially cubic perovskite structure, substantially stable in air over the temperature range of 25-950 °C.

US 6,090,500 relates to a novel rare earth gallate based oxide mixed conductive substance having a perovskite type structure. The membrane formed is a single bare membrane which could be used as air separation membrane.

WO2005/007595 describes a mixed (electronic and of anions O²⁻) conductive material having a perovskite structure which is used in catalytic reactor as air separation membrane for reforming methane or natural gases in synthetic gas (a mixture of H₂/CO). Thus this document shows that all the perovskite membranes could not be used to form C2 hydrocarbons from natural gases, especially from methane.

Application US2004/0101472 relates to a study performed regarding method for partial oxidation of methane. This document gives a solution to the problems revealed by the prior art, especially the decrease in oxygen supply from ceramic membrane and the catalyst consumption. The solution described is disposing a methane partial oxidation catalyst based on Ni or on noble metal such as Ru that as a low tendency of carbon precipitation in such an amount that deactivation due to oxidation does not occur, in a two-dimensional arrangement in the vicinity of the surface of the ceramic membrane where oxygen comes out thereof in the upstream of the methane containing gas flow in the oxygen permeation membrane reactor, and disposing the methane partial oxidation catalyst based on Ni or on noble metal, such as Ru that as a low tendency of carbon precipitation, in a 3-dimensional arrangement with respect to the ceramic membrane in the downstream of the methane-containing gas flow in the reactor.

A first study realized by the inventors (Haag et al., Catalysis Today, 2007, 127, 157-164) describes the influence of oxygen supply rates on performances of catalytic membrane reactors and the application to the oxidative coupling of methane. Different membranes were tested (BSCFO, BBFO and BSMFO) with or without a catalyst layer deposited on the membranes. It is shown that membrane surface modified by a catalyst layer improve the efficiency and enhance C2 selectivity and methane conversion. However, it is demonstrated that the membranes present some drawbacks, e.g. the BBFO membrane lacks of thermal stability and cracking or partial melting of the disk is observed increasing the temperature beyond that point. Moreover, the oxygen diffusion through BSMFO membrane is much more difficult compared to the other perovskite samples. A second study of the present inventors (Olivier et al., Catalysis Today, 2009, 142, 34-41) relates to the influence of the catalyst layer on a dense perovskite membrane (BSCFO) in the oxidative coupling of methane.

However, each of these membranes does not provide sufficient oxygen fluxes and/or efficient yield of C2 hydrocarbons for the OCM reaction. The main drawback is thus an insufficient oxygen fluxes in contact with the catalyst since some of the O²⁻ anions recombine to give O₂ which penalizes the selectivity over C2.

In order to extend the benefit of dense catalytic membrane reactor concept, the inventors have focused on providing a larger active surface area and better catalyst coating for a given geometry while establishing better control of the oxygen fluxes providing suitable catalytic performances.

A first objective of the invention is to provide a novel membrane exhibiting an improved oxygen connectivity.

Another objective of the invention is to provide such a membrane exhibiting an improved combination of oxygen availability and catalytic activity.

A still another objective of the present invention is to provide such a membrane, having enhanced properties (especially mechanical and thermal resistance, catalytic activity...), for high temperature oxygen permeation.

Another objective of the present invention is to provide a new way for carrying out catalytic partial oxidation reactions of carbohydrates in the temperature range of 800 to 1200 °C.

Another objective of the present invention is to provide a new way for the synthesis of C2 hydrocarbon via the oxidative coupling of methane reaction (OCM).

An objective of the present invention is also to provide a new way for carrying out those oxidations which is more efficient, especially in terms of yield.

The strategy provided by the present inventors to enhance the membrane properties, is based on the idea of employing a highly permeable dense membrane coupled to a thin porous layer of another mixed anionic-electronic conducting material with lower permeability.
A mixed anionic-electronic conducting material means that the material may conduct both ions (such as O²⁻) and electrons.

The drawbacks of the previous membranes, as described above, are raised by the present invention which concerns an oxygen permeable composite membrane comprising :
- a first dense perovskite membrane, and
- a porous perovskite membrane on one side of the dense perovskite membrane.

« Dense » is understood to mean a non-porous layer, that is to say a layer without macro, meso or micro pores, which enables to forbid the passage of a neutral gas. Only anionic species such as O²⁻ can pass through the membrane, moving from defect to defect in the structure with an inverse moving of the electrons to ensure the neutrality of the whole.

The dense perovskite membrane is intended to be placed in contact with the oxygenated part of the reactor and the porous membrane is intended to be placed in contact with the natural gases or higher hydrocarbons to be treated.

In one embodiment the membrane further comprises a catalyst layer at the surface of, preferably deposited onto, the porous membrane. This embodiment creates a sandwich membrane comprising a first dense perovskite membrane, a porous interlayer perovskite membrane on the dense perovskite membrane and a catalyst layer on the porous membrane.

In one embodiment, the porous membrane is deposited on the dense membrane. "Deposited" means that the material intended to form the porous membrane, respectively the catalyst layer, is placed at the surface of the dense membrane, respectively of the porous membrane, in a particulate form, in particular in suspension (liquid or gel), for example via a dip-coating method, a CVD method, a spray-drying method for the porous membrane, respectively a sol-gel method, a wash-coating method or a CVD method for the catalyst layer, and is then treated to form and attach the porous membrane on the dense membrane, respectively the catalyst layer on the porous membrane.
In a preferred embodiment, the porous membrane is sintered on the dense membrane.

The catalyst layer is thus intended to be placed in contact with the natural gas or higher hydrocarbons to be treated.

The membrane can have any shape and for example can be either in a disk form or in a tubular form.

In an embodiment, the dense perovskite membrane presents high oxygen permeability, for example from 1 to 3 mL.min⁻¹.cm⁻² at 950°C and high thermal stability. This membrane is used as the support membrane of the composite material of the present invention.

In an embodiment, the composite membrane is characterized in that the oxygen permeability of the porous perovskite membrane is lower than that of the dense perovskite membrane.

In an embodiment, the porous membrane is a macro-mesoporous membrane. According to a feature, the average pore size of the porous membrane is comprised between 10 and 1000 nm, preferably between 20 and 200 nm.

In an embodiment, the porous membrane is formed from a perovskite layer.

In one embodiment the layer forming the porous membrane is formed of agglomerated particles, in particular having a diameter size of between 10 and 80 µm, preferably between 20 and 50 µm.

Advantageously, the porous layer, for example sintered onto the dense membrane, shows slight porosity to oxygen anions, thus raising the specific surface area available for the catalytic action.

In one embodiment the ionic conductivity of the porous membrane is lesser than the conductivity of the dense membrane.

In one embodiment the ionic conductivity of the dense membrane is between 2 and 15 times greater than the conductivity of the porous membrane.

In one embodiment of the present invention, the whole thickness of the composite membrane made of the dense membrane and the porous membrane is comprised between 0.5 and 3 mm preferably between 1 and 2 mm.

In another embodiment of the present invention, the whole thickness of the sandwich membrane is comprised between 0.5 and 3.5 mm, preferably between 1 and 2 mm.

In one embodiment the thickness of the dense membrane is comprised between 0.4 and 2.5 mm preferably between 0.5 and 1.5 mm.

In another embodiment the thickness of the porous membrane is comprised between 50 and 500 µm, preferably between 150 and 300 µm.

In another embodiment the thickness of the catalyst layer, if present, is comprised between 10 and 200 µm, preferably between 20 and 100µm. In one embodiment the catalyst layer penetrates in part in the porous layer.

In one embodiment the catalyst layer thickness is lower than the porous membrane thickness. The maximum loading and therefore thickness are limited by the fragility of the porous membrane induced by its high porosity.

In one embodiment of the present invention, the first dense perovskite membrane is chosen among: a Ba_{0.5}Sr_{0.5}Co_{0.8}Fe_{0.2}O_{3-δ} (BSCFO) membrane, La_{0.2}Sr_{0.8}Co_{0.8}Fe_{0.2}O_{3-δ} (LSCFO), Ba_{0.6}Sr_{0.4}Zr_{0.7}Co_{0.2}Fe_{0.1}C_{3-δ} (BSZCFO) or of the BiMeVOx type.

In one embodiment the first dense perovskite membrane is a BSCFO membrane.

In one embodiment, the porous membrane is chosen among: a BaBi_{0.4}Fe_{0.6}O₃ (BFFO) membrane, La_{0.2}Sr_{0.8}Co_{0.8}Fe_{0.2}O_{3-δ} (LSCFO), Ba_{0.6}Sr_{0.4}Zr_{0.7}CO_{0.2}Fe_{0.1}O_{3-δ} (BSZCFO) or of the BiMeVOx type.

In one embodiment the porous membrane is a BaBi_{0.4}Fe_{0.6}O₃ (BFFO) membrane.

In one embodiment the first dense perovskite membrane is a BSCFO membrane and the porous membrane is a BFFO membrane.

Magnesium oxide is a well known catalyst support for the OCM reaction showing high thermal stability and no deep reducibility under the operating conditions. In general MgO is associated to Lithium (Li). However, membranes require high operating temperatures (800-1100 °C) to provide adapted oxygen permeability and due to its high volatility under those conditions Li was excluded as catalyst for the membrane of the invention. Platinum (Pt), which is much more resistant to high temperature and is known to easily activate methane in partial oxidation, may be used.

Calcium oxide is also a well known support for the OCM catalyses, since it is stable at high temperature under the reaction conditions. It is often associated to lanthanum (La) and strontium (Sr) as a good catalyst for OCM. Advantageously the La, Sr and Ca oxides and carbonates are thermally stable at calcination temperature and no sintering of the catalyst layer should occur.

In one embodiment, the catalyst is any type of OCM catalysts which do not comprise component volatile at temperature between 800 and 1200 °C. Preferably the catalyst is chosen between Pt/MgO, LaSr/CaO, Sr/La₂O₃ and V/MgO.

In one embodiment the catalyst is Pt/MgO or LaSr/CaO.

In one particular embodiment of the present invention the membrane comprises :
- a dense BSCFO membrane support,
- a porous BBFO interlayer sintered onto the BSCFO membrane, and
- a catalyst layer, wherein the catalyst is Pt/MgO or LaSr/CaO.

The composite membrane of the present invention has a hierarchical structure where the oxygen selective dense perovskite membrane exposes a very small specific surface area, enlarged by the porous membrane to be then further extended by the final catalytic membrane if present. Without being bound to theory, the higher specific surface area of the membrane of the present invention could explain the higher O₂ fluxes as the surface release of oxygen was previously identified (in the prior art) as rate limiting step in the permeation.

One important advantage of the hierarchical structure relates to the stability of the membrane. Indeed, performances are remaining stable under the harsh operating conditions of OCM, or other oxidative reactions enabling an oxygen flux at high temperature.

Advantageously the membrane of the present invention is stable even after several dozen days of reaction.

In one embodiment of the present invention, the composite membrane, comprising a dense perovskite membrane and a porous perovskite membrane, presents a specific surface area, on the porous phase, comprised between 50 and 1000 cm² g⁻¹ preferably between 100 and 500 cm² g-¹.

In one embodiment of the present invention, the sandwich membrane, according to the invention, comprising a catalyst layer, presents a specific surface area, on the catalyst phase, comprised between 1000 and 5000cm².g⁻¹, preferably between 1500 and 3000cm²_{.}g⁻¹.

A significant increase of the specific surface area of the composite membrane is observed, it may be typically around 1900 cm²/g for the membrane comprising the catalyst layer, and around 150 cm²/g for a BSCFO-BBFO membrane, whereas it is much more weaker for a single bare membrane (e.g.: a single bare BSCFO membrane).

In one embodiment the membrane according to the invention, comprising perovskite powder which is sintered on a dense perovskite membrane, have a roughly surface which enables a better catalyst adherence, the catalyst layer becoming partially immerged in the interlayer, thus providing a well interfaced system. This allows also one to use and include more catalyst. Typically, the increase of catalyst loading is 3 to 4 folds with respect to possible loading on the only dense membrane, which consists in a very important and satisfying improvement. Due to the surface roughness of the membrane, catalyst adherence at high temperature is well superior (around three times higher) than that obtained for bare dense perovskite membrane surfaces.

The improved adherence of the catalyst layer regarding the prior art is also due to the high mechanical stress that is induced inside the membrane structure by the thermal treatments requested for calcination and sintering of the three different layers.

The amount of catalyst on a composite membrane according to the present invention is higher than that of a single membrane.

In one embodiment the amount of catalyst on the membrane is of between 5 mg/cm² and 50 mg/cm², preferably between 10 and 20 mg/cm².

In one embodiment, the catalyst layer covers the whole membrane surface.

In one embodiment, the catalyst forms a relatively uniform layer even after heating at high temperature.

The catalyst layer could, as described below, be deposited by any known method in the art.

In one embodiment the catalyst is Pt/MgO catalyst.

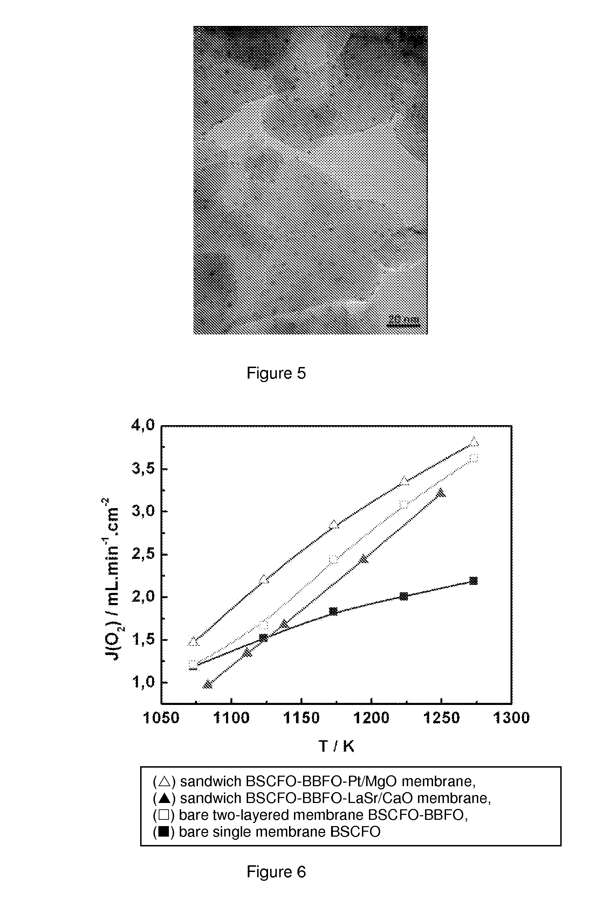

Advantageously, the membrane is characterized in that the platinum particles are very well dispersed on the whole Mg support (figure 5) and the diameter (solvated diameter) of the platinum particles is estimated at about 1.5 to 2 nm. Without to be bound by the theory, the efficiency of the catalyst depends on the number of Pt atoms on the surface, the larger the gas (methane)-active phase (Pt) interface the smaller the grains. Assuming spherical platinum particles, the dispersion, is approximately 70% as estimated by the correlation reported by Scholten et al (J.J.F. Scholten, A.P. Pijpers, A.M.L. Hustings, Catal. Rev. Sci. Eng., 1985, 27, 151-206).

In another embodiment the catalyst is LaSr/CaO catalyst. Advantageously the solvated diameter of the catalyst particles is comprised between 10 and 50 nm.

LaSr/CaO catalyst layer presents a high cristallinity. This enables especially an enhancement in stability.

The mobility of ionic oxygen in the catalyst layer (e.g: Pt/MgO, LaSr/CaO) is low, thus it seems highly desirable to establish a mean to distribute oxygen to a very thin layer of catalyst. This distribution is ensured by the porous layer allowing conducting oxygen from the dense membrane to the catalyst located at the surface of the porous membrane. The permeability to the oxygen anion of the porous membrane is lower than that of the dense perovskite membrane to avoid that the surface of the porous membrane allows extracting more oxygen from the membrane that what could be supplied across the dense perovskite membrane. Ionic oxygen is transferred from the dense membrane into the porous membrane desorbing from there across the enlarged surface area.

The catalyst performance depends crucially on the availability of oxygen. There is an optimisation of the amount of catalyst according to the anion oxygen flux. The specific surface area is enlarged thanks to the porous membrane which enables each oxygen anion to be available and well distributed for the catalyst.

It is utmost important to avoid a deep reduction of the perovskite membrane in order to avoid membrane failures.

« Deep reduction » is understood to mean a diminution of the average concentration of oxygen to the oxide to values lesser than the stoechiometry of the perovskite which lead to a phase change and to the destruction of the crystal lattice of the ABO₃ type.

The composite membrane of the present invention combines the advantages of defect-freeness and selectivity from a dense perovskite membrane, the developed specific surface of a porous perovskite system and the chemical stability of a composite material with very good catalytic performances.

Thus the composite membrane of the present invention enables better control of the oxygen fluxes through the two perovskite layers providing adequate activated oxygen to the catalyst, if present, during the reaction.

In one embodiment, the membrane of the present invention presents enhanced properties, especially enhanced oxygen permeability, flexibility, i.e., a better stability with respect to various gases mixtures which are more or less reductors or oxidants, thermal stability and catalytic properties.

In one embodiment, the membrane of the present invention presents an oxygen permeability of between 0.5 and 10 mL.min⁻¹.cm⁻², preferably between 1 and 3 mL.min⁻¹.cm⁻² at 950 °C.

In one embodiment, the membrane of the present invention is stable at high temperature especially up to about 1200 °C.

The present invention also concerns a method of production of the membranes described above.

The method of preparation of the membranes according to the present invention, comprised the following steps:
a) providing a perovskite by pressing perovskite powder,
b) densification of the perovskite membrane by sintering, preferably in air, at high temperature, especially between 1000 °C and 1200 °C,
c) providing a perovskite powder,
d) calcination of the perovskite powder, preferably in air, at high temperature, especially above 800 °C,
e) milling, especially ball-milling (i.e. milling until to obtain thin particles), of the powder in alcohol suspension to obtain particles,
f) deposition on one side of the dense perovskite membrane obtained in b) of the powder obtained in e),
g) sintering the composite membrane obtained in d), preferably in air.

The membrane can be of any shape, e.g in a disk form or in a tubular form.

In one embodiment, the deposition of the porous membrane can be carried out by any method known in the art, especially by dip-coating, spray drying or CVD.

In one embodiment of the present invention the method further comprises a step h) of deposition of a catalyst layer onto the membrane obtained in g) and a final step i) of calcinating of the composite membrane, preferably in the air, in order to provide a "sandwich" membrane comprising a first dense perovskite layer, a porous perovskite interlayer and a catalyst outer layer. The catalyst outer layer is free of carbonaceous residues.

In one embodiment the step of deposition of the catalyst layer can be carried out by any method known by the person of the art, e.g. wash-coating, sol-gel or CVD. The method will depend on the catalyst used; a man skilled in the art is capable of determining the best method to be used.

In one embodiment the catalyst layer is deposited via a wash-coating method, which is a well known method for the person skilled in the art. Wash coating involves the deposition of a catalyst-containing suspension on one surface. The following is an example of a wash-coating method: the suspension was based on aqueous slurry of the catalyst powder using additionally two organic agents: an organic binder enable to adjust viscosity of inorganic sol-gel, for example cellulose ethers, for example Tylose^{®} sold by Clariant, and acetic acid. The organic binder acting as binding agent, has a role to adjust the viscosity of suspension and improves initial adhesion on the membrane surface prior to calcination, acetic acid impacts on the dispersion of the particles and on the spreading behaviour of suspensions on the surface of membrane. Catalyst and organic binder (Tylose^{®}) powder were first dry-mixed and the mixture was added to a continuously stirred aqueous solution of acetic acid maintained at 40 °C for several minutes. The slurry was kept stirring at ambient temperature in a closed beaker to avoid water loss by evaporation during few days, e.g three days. A further step of aging is required to obtain a homogeneous suspension. One droplet of suspension was spread on the surface of the membrane and following membrane drying for 2 hours in nitrogen atmosphere at 120 °C calcination proceeded for 6 h in air at 900 °C. In one embodiment, the catalyst is Pt/MgO. In this embodiment, MgO was wash-coated on the membrane surface and Pt was added by impregnation using tetraamine platinum(II) nitrate as metal precursor.

The platinum loading of the MgO was about 0.2 to 1.5wt%, preferably about 0.5wt% with respect to the weight of MgO, and the gain in weight after deposition of the Pt/MgO catalyst was 16g/cm².

In another embodiment, the catalyst is LaSr/CaO; in this embodiment LaSr/CaO catalyst was first prepared by oxalate coprecipitation: oxalic acid is added to an aqueous solution of lanthanum, strontium and calcium nitrates. Then oxalates precipitate and the solution is dried and the powder is crushed and sieved to get a particle size around 50 to 100 µm, typically around 80µm. Then the catalyst is deposited on the surface of the porous layer membrane by wash-coating, as described above. Mass deposited of LaSr/CaO is about 10 to 15 mg/cm².

The perovskite membrane and the perovskite powder can be obtained by any method known in the art, especially by the so-called citrate methods, which is a well known method for the person skilled in the art. It is for example described by Z. Shao, W. Yang, Y. Cong, H. Dong, J. Tong, and G. Xiong, Investigation of the permeation behavior and stability of a Ba0.5Sr0.5Co0.8Fe0.2O3-[delta] oxygen membrane. Journal of Membrane Science, 2000. 172(1-2): p. 177-188.

In one embodiment, step b is a sintering step. Step b is carried out at a temperature below the melting temperature of the perovskite membrane. The temperature is advantageously comprised between 1000 and 1200 °C, preferably between 1150 and 1180 °C.

In one embodiment, the temperature of step d is lower than that of step b in order to sinter the powder layer without densification of the layer. The temperature is advantageously comprised between 900 and 1100, preferably between 1000 and 1100°C.

In one embodiment the temperature of step i is lower than that of step d. The temperature of step i is advantageously comprised between 800 and 1100 °C preferably between 900 and 1000 °C.

It is important that the heating temperature of each step to be lower than the melting point of the last layer deposited and lower than the temperature of the last heating step, in order to not deteriorate the composite structure.

In one embodiment, the perovskite membrane is a BSCFO membrane.

In one embodiment, the perovskite powder is BBFO powder.

The invention also concerns the membrane obtained by the process described above.

The present invention also concerns the use of the membranes according to the invention in partial oxidation reactions of carbohydrates, partial coating reactions of carbohydrates both at high temperature, especially at temperature between 800 and 1200 °C.

In one embodiment the reaction is chosen between the OCM reaction, the high temperature oxidation reactions such as oxy-reforming of methane and other hydrocarbons, partial oxidation of ammonia.

In one embodiment the reaction is OCM reaction.

The present invention also concerns a method for the production of C2 hydrocarbons by OCM reaction in a membrane reactor using the membrane according to the present invention.

The OCM reaction is a known reaction in the art and provides C2 hydrocarbons (ethylene and ethene) from methane and air.

Methane and optionally an inert gas, e.g. helium, is (are) fed in one compartment of the reactor. Air is fed in the other compartment of the reactor, the two compartments being separated by means of the membrane according to the invention. Ionic oxygen passes through the membrane and reacts at the surface of the porous perovskite membrane or, if present, at the surface of the catalyst layer to furnish C2 hydrocarbons (ethylene and ethene).

In one embodiment the reaction temperature is comprised between 700 and 1200 °C, preferably between 800 and 1000 °C.

In one embodiment, the methane supply flow rate is comprised between 50 and 500 cm³/cm²/min, preferably between 80 and 200 cm³/cm²/min.

In one embodiment the air supply rate is comprised between 50 and 500 cm³/cm²/min, preferably between 80 and 200 cm³/cm²/min.

In one embodiment the total pressure for both hydrocarbon and air compartments during the reaction is comprised between 1 and 2 bar, preferably between 1 and 1.5 bar.

In one embodiment the yield of C2 hydrocarbons is comprised between 5 and 30%, preferably between 10 and 25%

In one embodiment the ratio ethylene/ethane is comprised between 1 and 10, preferably between 5 and 8.

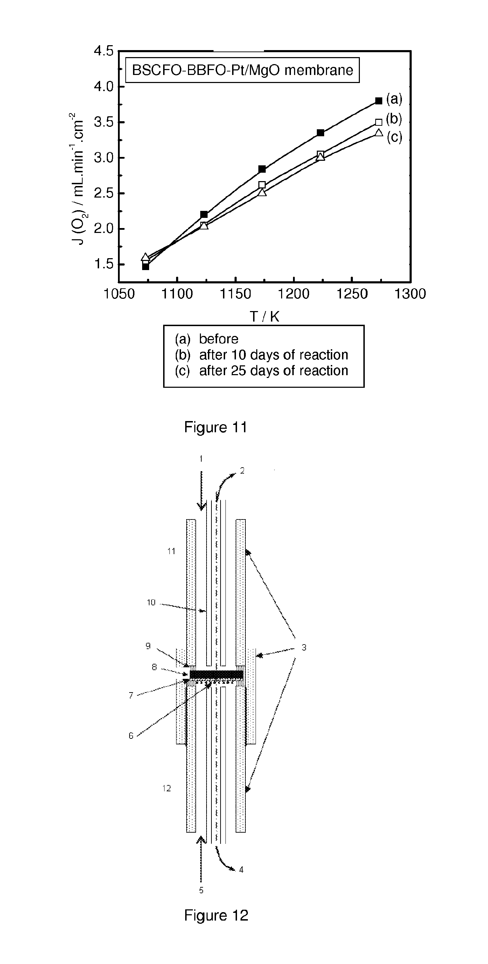

The invention also relates to a reactor, preferably a tubular reactor with two compartments separated by a composite membrane according to the invention, an example of which is represented on figure 12.

The invention will now be described in further details using the following non-limiting examples.

Figure 1 represents X-Ray Diffraction (XRD) patterns of the BSCFO membrane sintered at 1160 °C, the BSCFO-BBFO membrane calcined at 1040 °C and the BSCFO-BBFO-Pt/MgO « sandwich » membrane.

Figure 2a represents a microstructure view of the BSCFO-BBFO membrane.

Figure 2b represents a cross-section view of the BSCFO-BBFO membrane.

Figure 3 represents a microstructure view of the interface BBFO-LaSr/CaO.

Figure 4 represents a cross-section view of the sandwich membrane.

Figure 5 represents the dispersion of platinum on the MgO support by transmission electron microscopy (TEM).

Figure 6 represents oxygen permeation through different membranes (sandwich BSCFO-BBFO-Pt/MgO membrane, sandwich BSCFO-BBFO-LaSr/CaO membrane, bare two-layered membrane, bare single membrane) using a reconstituted air: 100 mL.min⁻/ He: 100 mL.min⁻¹ gradient; depending on temperature.

Figure 7 represents oxygen permeation through different membranes (BSCFO membrane, BBFO membrane and BSMFO membrane) using a reconstituted air: 100 mL.min⁻¹/He: 100 mL.min⁻¹ gradient; depending on temperature.

Figure 8 represents the catalytic activity (C2 productivity) of the BSCFO based membranes (BSCFO, BSCFO-BBFO, BSCFO-BBFO-Pt/MgO and BSCFO-BBFO-LaSr/CaO); depending on the temperature.

Figure 9 represents the catalytic activity of the sandwich membrane BSCFO-BBFO-Pt/MgO in function of methane concentration in the reactant feed, depending on the temperature.

Figure 10 represents a comparison of the C₂H₄/C₂H₆ ratio (with a methane concentration of 33.3% / reactant flow rate: 85 mL.min⁻¹) obtained with four different membranes (namely : BSCFO bare membrane, BSCFO-BBFO membrane, BSCFO-BBFO-Pt/MgO membrane and BSCFO-BBFO-LaSr/CaO membrane) depending on the temperature.

Figure 11 represents the stability of the BSCFO-BBFO-Pt/MgO membrane before and after 10 days of reaction and after 25 days of reaction according to the temperature reaction.

Figure 12 represents a reactor comprising the membrane of the present invention.

Example 1: Preparation of the membrane materials:
1 mm thick dense Ba_{0.5}Sr_{0.5}Co_{0.8}Fe_{0.2}O_{3-δ} (BSCFO) membrane disks were prepared according to specific conditions of preparation and densification reported elsewhere (S. Haag, A.C. van Veen, C. Mirodatos, Catal. Today, 2007, 127, 157-164M. Rebeilleau-Dassonneville, S. Rosini, A.C. van Veen, D. Farruseng, C. Mirodatos, Catal. Today, 2005, 104, 131). Briefly, the synthesis of the BSCFO oxide proceeded by the so-called citrate method using citric acid and EDTA as complex formation agents (EDTA was used to dissolve the barium nitrate). Citric acid was added in 1:1 molar ratio with respect to the sum of metal salts. After calcinations and milling of the resulting, a black colored perovskite powder membrane were prepared by isostatic pressing applying a pressure of 220 MPa for 2 minutes to the powder. Resulting disks were weighing ∼0.5 g, had a thickness of 1 mm and a diameter of 15 mm. Densification of the disk was achieved by sintering in air for 8 h at 1160°C with a heating and cooling ramps of 2°C/min. The final diameter of the disk had decreased to 13 mm by shrinkage during sintering.
BaBi_{0.4}Fe_{0.6}O₃ (BBFO) powder constituting the porous layer was also prepared according to the citrate method. Powders resulting from calcination in air at 900 °C during 4h were ball-milled in isopropyl alcohol suspension to obtain particles of about 10 µm. Then, following simple dip-coating of one side of the BSCFO disk, a BBFO layer was firmly attached to the membrane surface sintering the membranes in air at 1040 °C during 4h (ramps 2 °C/min). The amount of BBFO used to form the layer was about 30 mg in order to cover the whole surface of the selected side. As the sintering temperature was kept slightly below the BBFO melting temperature, BSCFO-BBFO membrane presents a considerable surface roughness, thus facilitating the following catalyst deposition on the intermediate layer.
Magnesium oxide was deposited by wash-coating and platinum was added by impregnation which is a well known method for the person skilled in the art, using tetraammine platinum (II) nitrate as metal precursor (Sigma-Aldrich) to yield finally the composite membrane.
The amount of platinum was estimated by chemical analysis (ICP/AES - Inductively Coupled Plasma / Atomic Emission Spectroscopy) using a Pt/MgO sample scraped from the membrane surface of reference samples prepared in parallel (followed by calcination of the sandwich membrane at 900 °C after deposition). The Pt loading of the MgO support was about 0.5 wt.-% and the gain in weight after deposition of the Pt/MgO catalyst was 16 mg/cm².
In the case of LaSr/CaO catalyst: 8 %wt of LaSr/CaO catalyst was prepared by oxalate coprecipitation: oxalic acid is added to an aqueous solution of La, Sr and Ca nitrates. Oxalates precipitate, the solution is then dried and the powder is calcinated in air at 850 °C for 6 h. The resulting powder is crushed and sieved to get a particle size around 80 µm. Then the catalyst is deposited on the surface of the BBFO layer of the membrane by wash-coating. Mass deposited is about 10 to 15 mg.

### Example 2 : Membrane characterization

### Structure :

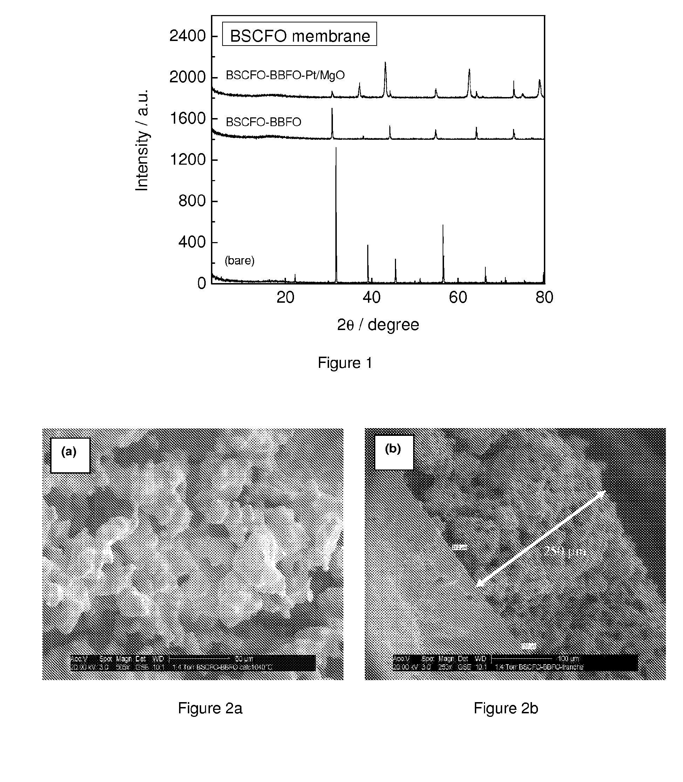

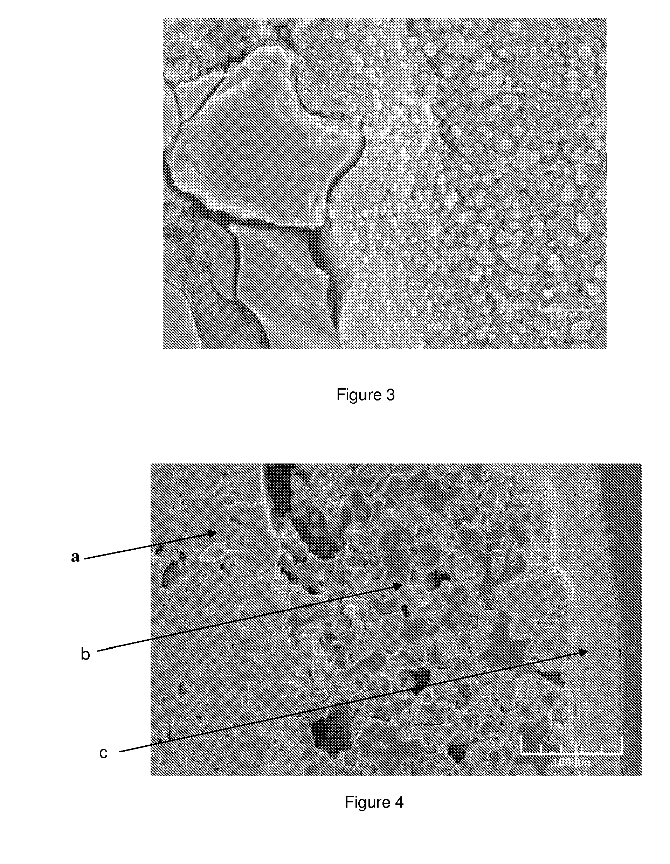

The structure of the membrane after the different steps of preparation was observed by X-ray diffraction, using a Bruker D5005 system in the 2θ range 3-80°, a step width of 0.02° and a counting time of 1s and Cu K_{α1+α2} radiation (1.54184 Å). The formation of the dense surface morphology required for a membrane was monitored by scanning electron microscopy (Hitachi S800) as well as respective micrographs of cross-sectional cuts allowed an estimation of the BBFO layer thickness.
The X-Ray diffraction patterns of the three different membranes of example 1 (bare BSCFO, BSCFO-BBFO and "sandwich" BSCFO-BBFO-catalyst) observed after calcination are presented in Figure 1. The desired perovskite phase (JCPDS: 00-055-0563) (JCPDS: Joint Committee on Powder Diffraction Standards) is detected for the bare BSCFO membrane. Regarding the BSCFO-BBFO membrane after the sintering of the BBFO powder on the BSCFO surface at 1040 °C, only the BBFO perovskite phase (JCPDS: 01-089-8403) is observed. Moreover, this phase is also detected for the sandwich membrane carrying a Pt/MgO catalyst after the final calcination step at 900 °C, while in this case the MgO periclase phase (JCPDS: 01-071-3631) is also observed for the latter sample. It should be noted that in good agreement with a high dispersion reported in the following no reflexes are observed for Pt.
The morphology of the BSCFO membrane (bare) was already depicted in previous works (S. Haag, M. Bosomoiu, A.C. van Veen, C. Mirodatos, in Studies in Surf. Catal. Vol. 167 (ed. F.B. Noronha, M. Schmal and E.F. Sousa-Aguiar) (2007), 19-24; S. Haag, A.C. van Veen, C. Mirodatos, Catal. Today, 2007, 127, 157-164) allowing to restrict the current description to microstructures of BSCFO-BBFO and BSCFO-BBFO-Pt/MgO membranes. Obviously the BBFO layer possesses an open macroscopic porosity, which indeed gives the surface of the BSCFO-BBFO membrane a certain roughness (Figure 2.a), but the BBFO layer seems to remain relatively uniform all across the membrane surface. The thickness of the BBFO layer attached to the BSCFO support is estimated to be about 250 µm (Figure. 2.b). Purely at the macroscopic level, this surface roughness allows better adherence of the coated catalyst becoming partially immerged in the BBFO layer, thus leading to a well interfaced system. The catalyst layer itself covers the whole membrane surface (figures 3) and scanning electron microscopy shows a relatively uniform layer even after the final calcination step at 900 °C.
Figure 3 represents a cross section view of the interface BBFO-LaSr/CaO. The porous membrane is on the left side of the photography and the catalyst layer is on the right side.
Figure 4 represents a cross section view of the sandwich membrane wherein part a represents the dense perovskite membrane, part b represents the porous layer and part c represents the catalyst layer.

### Specific surface area:

Furthermore, the specific surface areas of the different membranes (bare BSCFO, BSCFO-BBFO and "sandwich" BSCFO-BBFO-catalyst) were evaluated by the BET method placing an entire membrane disk in a specific measurement cell to fit to a micrometrics ASAP 2020 apparatus. In the present case, krypton appeared as more appropriate adsorbent than nitrogen as it allowed reasonable precisions for set of membranes exhibiting only very small absolute surface areas. The results of the measurement of the surface area (in cm²/g) of the three different membranes are presented in table 1.

**Table 1 : Estimation of specific surface areas for BSCFO based membranes (bare, BSCFO-BBFO and BSCFO-BBFO-Pt/MgO)**

| **Membranes** | **BSCFO (bare)** | **BSCFO-BBFO** | **BSCFO-BBFO-Pt/MgO** |
|---|---|---|---|
| **Specific surface area (cm².g⁻¹)** | **< detection limit** | **154** | **1947** |

Table 1 gathers the results on surface areas for each membrane. The standard deviation of each BET measurement is ±5 cm².g⁻¹ for the used experimental setup.
BET measurements reveal higher specific surface area for BSCFO-BBFO membranes compared to the (bare) dense BSCFO membranes. This is in perfect agreement with a surface enhancement effect of coatings, and is indeed expected given the porous character of the perovskite layer as revealed by microscopic cross-section views outlined formerly. Focussing on the impact of the Pt/MgO catalyst deposition, a significant increase of the specific surface area is observed for the sandwich membrane (∼ 1950 cm²/g) compared to the BSCFO-BBFO membrane (∼ 150 cm²/g).
In summary it should be noticed that the exposed surface of the sandwich membrane has a hierarchical structure where the oxygen selective BSCFO disk exposes a very small surface, enlarged by the BBFO layer to be then even further extended for the final catalytic membrane carrying the OCM catalyst.

### Dispersion of platinum and morphology of the LaSr/CaO:

Transmission electron microscopy, on a JEOL 2010 instrument, served to establish the dispersion of platinum on the MgO support (figure 5) and the morphology of the LaSr/CaO catalyst on the surface of the membrane (figure 3).Pt/MgO powder collected by scraping from the sandwich membrane surface was used to study the dispersion of the platinum on the MgO support with transmission electron microscopy (see figure 5). Figure 5 represents the dispersion of platinum on the MgO support. The distribution of platinum on the MgO is homogeneous.
The platinum particles are very well dispersed on the whole MgO support while the diameter of the platinum particles is estimated at about 1.5 to 2 nm.
Assuming spherical platinum particles, the dispersion is approximately 70% as estimated by the correlation reported by Scholten et al. [J.J.F. Scholten, A.P. Pijpers, A.M.L. Hustings, Catal. Rev. Sci. Eng., 27 (1985), 151-206].
SEM also shows the high cristallinity of the LaSr/CaO catalyst layer deposited on the BBFO (figure 3) before reaction and also the high porosity of this layer. The initial diameter of catalyst particles put in suspension before coating is about 80 µm, however, SEM pictures do not allow determining any catalyst particle size, whereas it is possible for the sintered BBFO layer.

### Example 3: Oxygen permeability

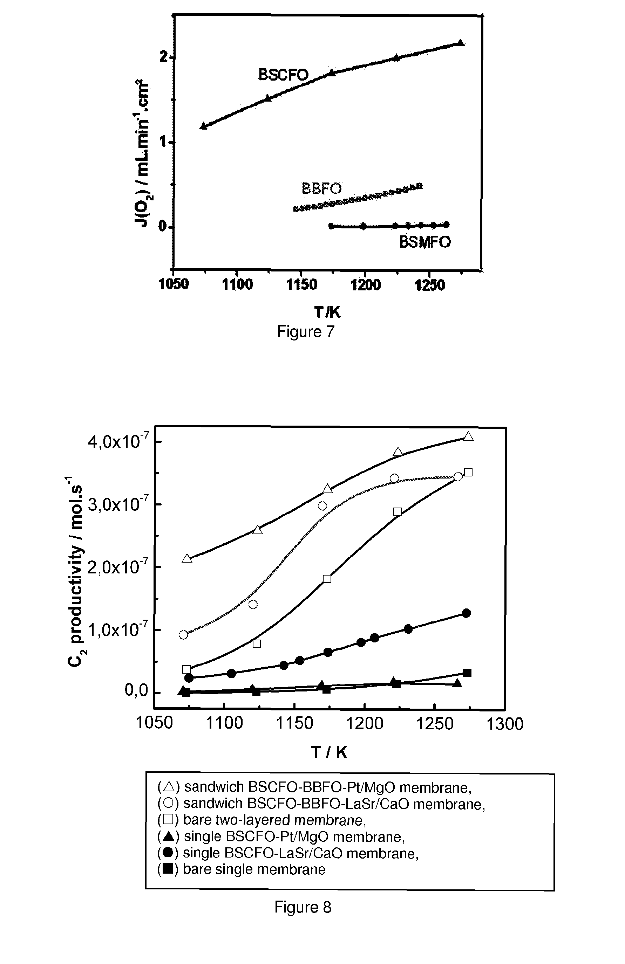

The oxygen permeability was acquired in the temperature range from 800 °C to 1000 °C. An O₂/N₂ mixture presenting reconstituted air was fed to the oxygen-rich membrane compartment, while helium was used as a sweep gas on the permeate side. The experimental device and the sealing process were detailed in previous work (S. Haag, A.C. van Veen, C. Mirodatos, Catal. Today, 2007, 127, 157-164). The total pressure at the oxygen-rich side was adjusted to 1.1 bar and a constant total flow rate of the mixed O₂ and N₂ streams was controlled by mass flow controllers.
Gas chromatography (HP 5890 Series II, 13X packed column) allowed complete analysis of gases at both sides of the membrane.
The oxygen permeation fluxes for the BSCFO (bare), the BSCFO-BBFO, the "sandwich" BSCFO-BBFO-Pt/MgO, the "sandwich" BSCFO-BBFO-LaSr/CaO membranes, the BBFO membrane and the BSMFO membrane are presented in figures 6 and 7.
As expected, higher oxygen permeation rates are observed for the BSCFO-BBFO and sandwich BSCFO-BBFO-Pt/MgO and BSCFO-BBFO-LaSr/CaO membranes compared to the bare BSCFO, BBFO and BSMFO membranes.
In fact, the oxygen flux is about 3mL.min⁻¹.cm⁻² at 950°C (1223K) for the BSCFO-BBFO membrane while it is around 2mL.min⁻¹.cm⁻² for the bare BSCFO membrane. However, the oxygen permeability of the sandwich LaSr/CaO membrane is even lower than the one of the single bare BSCFO membrane, at temperatures lower than 850 °C (1123K), while higher at temperatures above 850 °C (1123K).
Higher specific surface areas of the BSCFO-BBFO and BSCFO-BBFO-Pt/MgO membranes could explain the higher O₂ fluxes as the surface release of oxygen was previously identified as rate limiting step in the permeation (S. Haag, A.C. van Veen, C. Mirodatos, Catal. Today, 2007, 127, 157-164).
On the other hand, given the lower permeability of the BBFO material constituting the porous distribution layer, this observation obviously underlines that ionic oxygen is successfully transferred from the BSCFO bulk material into the BBFO layer desorbing from there across the enlarged surface area. Thus, the higher permeation rate observed for the BSCFO-BBFO membrane underlines that the distribution to the larger BBFO surface proceeds indeed efficiently and that a catalyst located on the interface with the BBFO would be more homogeneously supplied that it would be the case with a catalyst just interfacing the small BSCFO surface.
Regarding the BSCFO-BBFO-Pt/MgO case, it has already been demonstrated that the addition of the Pt/MgO layer enhances the oxygen permeation through the composite membranes (S. Haag, M. Bosomoiu, A.C. van Veen, C. Mirodatos, in Studies in Surf. Catal. Vol. 167 (ed. F.B. Noronha, M. Schmal and E.F. Sousa-Aguiar) (2007), 19-24; S. Haag, A.C. van Veen, C. Mirodatos, Catal. Today, 2007, 127, 157-164).
Accounting for the surface areas, the BBFO layer seems to slow down the oxygen permeation through the composite membranes, by adding another step to the oxygen transport before meeting the metal supported catalytic layer. This study underlines the crucial role of the intermediate BBFO layer supplying the activated oxygen to the catalyst during the reaction.
Therefore, the additional step in the oxygen transport through the composite membrane should allow appropriate oxygen fluxes to the catalytic formulation, which should drive the membrane reactor to better catalytic performances.

### Example 4: Comparison of the catalytic performances of the different membranes

The experimental setup for the catalytic tests with membrane reactors was also described in previous work (S. Haag, A.C. van Veen, C. Mirodatos, Catal. Today, 2007, 127, 157-164). Oxygen from the air feed supplied to the mixed conducting membrane crossed selectively the dense perovskite disk and acted as activated species on the catalyst surface in the oxidative coupling of methane. The CH₄/He mixture flow rate was fixed to 85 mL.min⁻¹ varying the methane concentration in the reactant feed and the reconstituted air flow rate was set to 100 mL.min⁻¹. The experiments were conducted in the temperature range of 800 to 1000 °C.
Figure 8 reports the catalytic performances of the different membrane reactors for experiments with a methane concentration of 10%, in 85 mL.min⁻¹ reactant feed on the reactant side while on the other side of the membrane a flow of 100 mL.min⁻¹ of reconstituted air was used.
According to figure 8 the BSCFO bare membrane shows the lowest C₂ productivity in comparison to the modified membranes. The two-layered membrane (BSCFO-BBFO) enables an enhancement of the C2 productivity compared to the single bare BSCFO membrane or to the BSCFO-catalyst membrane, especially at higher temperature.
The sandwich membranes (BSCFO-BBFO-catalyst) enable an enhancement of the C2 productivity. When the catalyst is Pt/MgO, the C2 productivity is more than 2 times greater than that of the bare membrane and the BSCFO-catalyst membrane at lower temperature and more than 4 times at higher temperature. This result is especially remarkable considering the fact that Pt/MgO powders used in a conventional fixed bed reactor for the oxidative coupling of methane reaction present very low C₂ selectivity and productivity, driving preferentially the reaction to the production of synthetic gas and additional carbon dioxide.
When the catalyst is LaSr/CaO, the C2 productivity is more than 3 times greater than that of the bare membrane and the BSCFO-catalyst at higher temperature.
On the other hand, one of the most promising results is the increasing of the C₂ productivity with the sandwich membrane when increasing the methane concentration in the feed outlined in figure 8. For example, the C₂ productivity rises at 950°C (1223K) from 3.83.10⁻⁷ mol.s⁻¹ with a 10% methane concentration in the reactant feed to 1.1.10⁻⁶ mol.s⁻¹ with a 33.3% CH₄ concentration in the reactant feed.

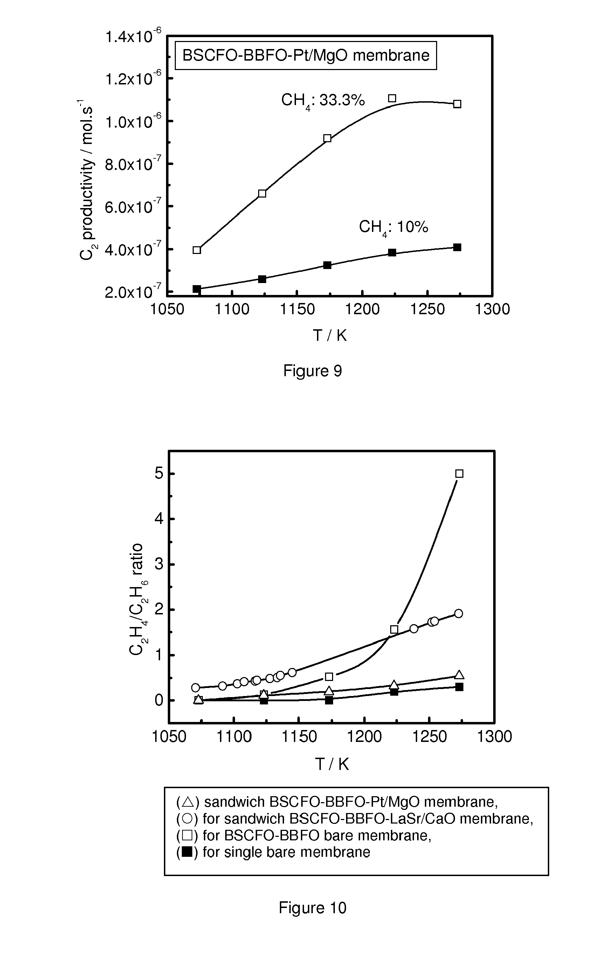

Figure 10 represents a comparison of the C₂H₄/C₂H₆ ratio obtained with the different membranes.
The single bare membrane presents the lowest C2 selectivity.
At lower temperature the sandwich membranes (BSCFO-BBFO-Pt/MgO and BSCFO-BBFO-LaSr/CaO) and the BSCFO-BBFO membrane present an enhance C2 selectivity compared to the single bare membrane.
At higher temperature, the BSCFO-BBFO-Pt/MgO membrane presents a greater selectivity than the single bare membrane.
At higher temperature the BSCFO-BBFO-LaSr/CaO membrane presents a C2 selectivity more than 1.5 times greater than that of the single bare membrane.
At higher temperature the BSCFO-BBFO membrane presents the highest C2 selectivity which is more than 5 times greater than that of the single bare membrane.
With those different assays, the man skilled in the art will be able to choose the best membrane between double-layered membrane or sandwich membrane, and the best catalyst, according to the product he wants. The double-layered membrane enables to have a great C2 selectivity but a lesser productivity. On the contrary, sandwich membrane enables to have great productivity but lesser selectivity.
It may be concluded that the BBFO layer plays also an important catalytic role while enhancing both, methane conversion and C₂ selectivity, compared to what is obtained with the BSCFO bare membrane.
On the other hand, the performance enhancement is much more important with increasing temperatures as the C₂ production increases much faster with the modified membranes than with the bare membrane. Hence, the intermediate layer of the sandwich membrane acts not only as an oxygen provider but possesses also catalytic activity. However, given the strong performance enhancement with Pt/MgO deposition, catalyst addition still remains an important factor for improving the catalytic performances of the membrane reactor.
The results obtained with this new kind of sandwich membrane can be compared to those reported in the literature for disk membranes used in a membrane reactor for the oxidative coupling of methane (Z. Shao, H. Dong, G. Xiong, Y. Cong, W. Yang, J. of Mem. Sci., 183 (2001) 181; (ref 11) J. Han, Y. Zeng, Y.S. Lin, J. Mem. Sci., 132 (1997) 235; (ref 12) V.V. Kharton, A.A. Yaremchenko, E.V. Tsipis, A.A. Valente, M.V. Patrakeev, A.L. Shaula, J.R. Frade, J. Rocha, Appl. Catal. A : Gen., 261 (2004) 25; (ref 13) J.E. ten Elshof, H.J.M. Boumeester, H. Verweij, , Appl. Catal. A : Gen., 130 (1995) 195; Y. Zeng, Y.S. Lin, S.L. Swartz, J. Mem. Sci., 150 (1998) 87). These results are presented in following Table 2.

**Table 2: Typical values of C₂ productivities reported in the literature for disk-shaped membrane reactors**

| **Membrane** | | **T** / °**C** | **Catalytic results** | | | **Ref.** |
|---|---|---|---|---|---|---|
| **Type** | **Geom. /mm** | | **Catalys t** | **C₂ prod.** / **µmol.s⁻¹.cm⁻²** | **S(C₂)/ %** | |
| *BSCFO-BBFO* | *T: 1.5* Ø: *7* | *800-1000* | *Pt*/*MgO* | 2.2 (950°C) | *32-50* | *This work* |
| *BSCFO-BBFO* | *T: 1.25* Ø: *7* | *800-1000* | *No* | *1.47* (950°C) | *38-60* | *This work* |
| *BSCFO (bare)* | *T: 1* Ø: *7* | *800-1000* | *No* | *0.0672* (950°C) | *2-10* | *This work* |
| *Bi_{1,5-x}Y_{0,5}CuₓO₃ 0,005<x<0,015* | *T: 1-3* Ø: *25,5* | *650-850* | *Memb rane has failed as a catalyst* | | | *[11]* |
| *La₂Ni_{0,9}Cu_{0,1}O₃* | *T: 0,6-1* Ø: *9* | *800-900* | *Pt* | *.0011 (850°C)* | *C_{Z}H₄:0,16 C₂H₆ : 0,57* | *[12]* |
| *La_{0,6}Sr_{0,4}Co_{0,8}Fe_{0,2}O₃* | *T: 0,5-2* Ø*: 15,2* | *790-940* | *No* | *0.0018 (880°C)* | *C₂H₄:31-15 C₂H₆:32-30* | *[13]* |
| *Ba_{0,5}Sr_{0,5}Co_{0,8}Fe_{0,2}O₃* | *T:* Ø*: 17,1* | *800-900* | *No* | *0.0698 (850°C)* | *40-70* | *[3]* |
| *La_{0,8}Sr_{0,2}Co_{0,6}Fe_{0,4}O₃* | *T: 1,85* Ø: *7* | *650-950* | *No* | *0.38 (900°C)* | *40-70* | *[14]* |

The new BSCFO-BBFO based membrane exhibits by far the best results for disk shaped membrane reactors with excellent C₂ productivities.
Several studies have been performed using tubular membrane reactors for the oxidative coupling of methane. Wang et al. used dense membrane tubes of Ba_{0.5}Sr_{0.5}Co_{0.8}Fe_{0.2}O_{3-δ}
(S=1.599 cm² and 2.282 cm²) with a La-Sr/CaO powder as fixed bed catalyst and obtained same order of magnitude results in C₂ productivities compared to our 0.5024 cm² disk membrane. In fact, the tubular configuration exposes much larger active surface.

### Example 5 : Study of the membrane stability

Another major advantage of the composite membrane is its high stability under harsh operating conditions, especially compared to the unstable La_{0.8}Sr_{0.2}CO_{0.6}Fe_{0.4}O_{3-δ} membrane. Thus, repeated permeation tests have been used to evaluate the stability of the membrane after reaction. Figure 11 presents the results of the oxygen permeation using the BSCFO-BBFO-Pt/MgO membrane after 10 days of reaction and after 25 days of reaction. It was obvious that the membrane is still dense with only a decline in permeability at 950°C (1123K) of about 9% after 10 days of reaction and of about 10% after 25 days of reaction compared to the freshly mounted sample. Therefore, even at high methane concentrations in the reactant feed and operation in the temperature range from 800 to 1000°C (1073 to 1273K) during OCM reaction, the new composite membrane remains stable, which is remarkable considering that dense bulk BBFO membranes are found to be not stable under these operating conditions (S. Haag, M. Bosomoiu, A.C. van Veen, C. Mirodatos, in Studies in Surf. Catal. Vol. 167 (ed. F.B. Noronha, M. Schmal and E.F. Sousa-Aguiar) (2007), 19-24; S. Haag, A.C. van Veen, C. Mirodatos, Catal. Today, 2007, 127, 157-164).

### Example 6 : Reactor comprising the membrane of the invention

Figure 12 represents a reactor comprising the sandwiched membrane of the present invention intended to be used for the OCM reaction. (1) represents the inlet of the mixture N₂+O₂, (2) represents the outlet of the mixture N₂+O₂, (3) represents Al₂O₃ tubes, i.e. the walls of the reactor, (4) represents the outlet of the products of the reaction, (5) represents the inlet of the mixture of methane and helium, (6) represents the catalyst layer constituting the sandwiched membrane, (7) represents the porous layer constituting the sandwiched membrane, (8) represents the dense perovskite layer constituting the sandwiched membrane, (9) represents the gold seal which enables the sealing of the 2 compartments (no air can pass), (10) represents quartz tube wich enables to separate the inlet of gases and the outlet of the product, (11) and (12) represent the two tubes forming the reactor.

## Claims

1. An oxygen permeable composite membrane comprising :
- a first dense perovskite membrane, and
- a porous perovskite membrane on one side of the dense perovskite membrane.

2. Membrane according to claim 1, wherein the porous perovskite membrane is deposited onto the dense perovskite membrane.

3. Membrane according to anyone one of claim 1 or 2 further comprising a catalyst layer on the surface of the porous membrane.

4. Membrane according to anyone of the preceding claims wherein the oxygen permeability of the porous perovskite membrane is lower than that of the dense perovskite membrane.

5. Membrane according to anyone of the preceding claims wherein the porous membrane is a mesoporous membrane.

6. Membrane according to claim 5 wherein the average pore size of the porous membrane is comprised between 10 and 1000 nm, preferably between 20 and 200nm.

7. Membrane according to anyone of the preceding claims wherein the thickness of the dense membrane is comprised between 0.4 and 2.5mm, preferably between 0.5 and 1.5mm.

8. Membrane according to anyone of the preceding claims wherein the thickness of the porous membrane is comprised between 50 and 500µm, preferably between 150 and 300µm.

9. Membrane according to anyone of the claims 3 to 8, which comprises a catalyst layer deposited on the porous layer, wherein the thickness of the catalyst layer is comprised between 10 and 200µm, preferably between 20 and 100µm.

10. Membrane according to anyone of the preceding claims wherein the dense perovskite membrane is a Ba_{0.5}Sr_{0.5}Co_{0.8}Fe_{0.2}O_{3-δ} (BSCFO), La_{0.2}Sr_{0.8}Co_{0.8}Fe_{0.2}O_{3-δ} (LSCFO), Ba_{0.6}Sr_{0.4}Zr_{0.7}Co_{0.2}Fe_{0.1}O_{3-δ} (BSZCFO) or of the BiMeVOx type membrane.

11. Membrane according to anyone of the preceding claims wherein the porous membrane is a BaBi_{0.4}Fe_{0.6}O₃ (BFFO), La_{0.2}Sr_{0.8}Co_{0.8}Fe_{0.2}O_{3-δ} (LSCFO), Ba_{0.6}Sr_{0.4}Zr_{0.7}Co_{0.2}Fe_{0.1}O_{3-δ} (BSZCFO) or of the BiMeVOx type membrane.

12. Membrane according to anyone of claims 3 to 11 wherein the catalyst is Pt/MgO, LaSr/CaO, SrLa₂O₃ or V/MgO.

13. Membrane according to anyone of claims 3 to 12 wherein it comprises:
- a dense BSCFO membrane support,
- a porous BBFO interlayer sintered onto the BSCFO membrane,
- a catalyst layer, wherein the catalyst is Pt/MgO or LaSr/CaO.

14. Membrane according to anyone of claim 1 or 2 wherein the specific surface area is comprised between 50 and 1000 cm².g⁻¹, preferably between 100 and 500 cm².g⁻¹.

15. Membrane according to claim 3 wherein the specific surface area is comprised between 1000 and 5000 cm².g⁻¹, preferably between 1500 and 3000 cm².g⁻¹.

16. Method for the preparation of a membrane according to anyone of claims 1 to 15 comprises the step of:
a. providing a perovskite by pressing perovskite powder,
b. densification of the perovskite membrane by sintering at high temperature,
c. providing a perovskite powder,
d. calcination of the perovskite powder at high temperature,
e. milling of the powder in alcohol suspension to obtain particles,
f. deposition, on one side of the dense perovskite membrane obtained in b), the powder obtained in e),
g. sintering the composite membrane obtained in d).

17. Method according to claim 16 wherein it further comprises a step h) of deposition of a catalyst layer onto the membrane obtained in g) and a final step i) of sintering the composite membrane in the air.

18. Method according to claim 17 wherein the catalyst is deposited by a method chosen between wash-coating, sol-gel or CVD methods.

19. Method according to anyone of the claims 16 to 18 wherein step b) is carried out at a temperature below the melting temperature of the perovskite membrane, preferably between 1000 °C and 1200 °C, preferably between 1150 and 1180 °C.

20. Method according to anyone of the claims 16 to 19 wherein step d) is carried out at a temperature lower than that of step b), preferably between 900 and 1100°C, preferably between 1000 and 1100 °C.

21. Method according to anyone of claims 17 to 20 wherein step i) is carried out at a temperature lower than that of step d), preferably between 800 and 1100 °C, preferably between 900 and 1000°C.

22. Use of the membrane according to anyone of claims 1 to 15 in a membrane reactor for catalytic partial oxidation reactions of carbohydrates in the temperature range of 800 to 1200 °C.

23. Use according to claim 22 wherein the reaction is the oxidative coupling of methane, oxy-reforming of methane or other hydrocarbons or partial oxidation of ammonia.

24. Method for the production of C2 hydrocarbons via the OCM reaction, wherein the reaction is carried out in a membrane reactor containing the membrane according to anyone of claims 1 to 15.

25. Reactor comprising 2 compartments separated by the composite membrane as claimed in claims 1 to 15.
